(19) Europäisches Patentamt
European Patent Office
Office européen des brevets

(11) **EP 4 324 452 A1**

(12) **EUROPEAN PATENT APPLICATION**

(43) Date of publication:
**21.02.2024 Bulletin 2024/08**

(21) Application number: **22306243.1**

(22) Date of filing: **19.08.2022**

(51) International Patent Classification (IPC):
**A61K 8/37** *(2006.01)*    **A61Q 1/14** *(2006.01)*
**A61Q 19/10** *(2006.01)*

(52) Cooperative Patent Classification (CPC):
**A61K 8/37; A61K 8/375; A61Q 1/14; A61Q 19/10;**
A61K 2800/5922

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Johnson & Johnson Consumer Inc.
Skillman, NJ 08558 (US)**

(72) Inventors:
• **Boucher, Amélie
  27430 ST ETIENNE DU VAUVRAY (FR)**
• **Fréville, Vianney
  27400 LOUVIERS (FR)**
• **Guyomard, Alexandre
  75013 PARIS (FR)**
• **Clémence, Hétroy
  60120 BRETEUIL (FR)**

(74) Representative: **Carpmaels & Ransford LLP
One Southampton Row
London WC1B 5HA (GB)**

(54) **IMPROVED CLEANSING COMPOSITION**

(57)    An emollient composition, a cleansing composition comprising the emollient composition, and a method of using a cleansing composition, the emollient composition including a blend of three different emollients, the emollients including an alkyl ester of myristate, an alkyl ester of cocoate, and a glycol ester of a fatty acid. The cleansing composition can remove at least one of mascara, lipstick and foundation from the surface of skin.

EP 4 324 452 A1

**Description**

BACKGROUND OF THE INVENTION

Field

The present invention relates to improved emollient compositions and cleansing compositions comprising said emollient compositions, for use in cleansing skin surfaces. In particular, the invention is useful in cleansing the facial areas of the skin, and is particularly useful in cleansing (that is, removing) various makeup products.

Background

Because of the wide variety of skin, hair and nail problems faced by consumers, consumers have long sought personal care products which can cleanse the skin, or deliver and/or deposit benefit agents that alleviate such problems. A facial cleanser should desirably provide a suitable cleansing efficiency for removal of makeup products, such as foundation, mascara and eyeliner. It is important, however, to avoid sacrificing the ability of products to adequately cleanse the skin in favor of reducing irritation to a user. Many delivery systems sacrifice aesthetics and cleaning ability in order to achieve stability and reduced irritation. This is particularly true when such products are to be used on sensitive areas, such as on the face, and even more particularly, in the very sensitive regions surrounding the eyes. It is beneficial that the cleansing product be capable of being applied by a product, such as a wipe, sponge, or applicator, or be applied by the hands directly.

Accordingly, it would be desirable to create such a composition that is capable of cleansing the skin of a user adequately.

SUMMARY

According to the present invention there is provided an emollient composition comprising:

    A. a glycol ester of a fatty acid;
    B. an alkyl ester of myristate; and
    C. an alkyl ester of cocoate.

According to the present invention there is provided a cleansing composition comprising the emollient composition as defined herein and a surfactant.

According to the present invention there is also provided the use of a cleansing composition as defined herein, or the use of an emollient composition as defined herein, to remove at least one of mascara, lipstick, and foundation from the surface of skin. In particular, the cleansing composition or the emollient composition may be used to remove lipstick.

DETAILED DESCRIPTION

The present invention provides emollient compositions and cleansing compositions that deliver optimal properties. As used herein, the term "optimal" includes comparable or improved cleansing efficacy when compared to known cleansers. Optimal does not necessarily mean 100% cleansing efficiency, but rather is a suitable efficiency to remove a sufficient amount of one or more cosmetics (that is, makeup products) from the face. Efficient cleansing is described below in greater detail for various cosmetics. Other benefits provided by the emollient compositions and/or cleansing compositions of the present invention may include one or more of the following: aesthetics, reduced greasiness, low irritation of skin and eyes, achieving shorter drying time with less force and lower cost, reducing the amount of cleansing agents in the composition, and enabling the emollient compositions and cleansing compositions to be free of silicone. As such, the emollient composition and/or the cleansing composition may be free of silicone. Additionally, or alternatively, the emollient composition and/or the cleansing composition of the present invention may be free of polyisobutenyl succinic anhydride derived emulsifiers or polyisobutylene derived emulsifiers. Additionally, or alternatively, the emollient composition and/or the cleansing composition may be free from preservatives, in particular, cleansing compositions having a low water content, such as having from about 1% to about 10% by weight of water may be free from preservative. As used herein, by "free of" a component is meant that the emollient composition and/or the cleansing composition has about 0.5% by weight or less, preferably about 0.1% by weight or less, preferably about 0.05% by weight or less of the component. More preferably, the emollient composition and/or the cleansing composition contains none of the component.

The emollient composition and/or the cleansing composition may contain only components that are derived from naturally occurring compounds. Additionally, the emollient composition and/or the cleansing composition may contain only components that are vegetal in origin. As such, the emollient composition and/or the cleansing composition may be free of components that are not vegetal in origin.

As used herein, the term "percent" shall refer to the weight percent, and similarly, all amounts set forth herein shall be by weight unless noted otherwise. As also used herein, the term "water dispersible component" shall mean a material that produces a uniform, clear or hazy, mixture when combined with at least a weight equivalent of water. The term "benefit agent" used herein includes any active ingredient that is to be delivered into and/or onto the skin, hair or nail at a desired location, including but not limited to agents such as a cosmetic agent or a pharmaceutical agent. By "cosmetic agent," it is meant any ingredient that is appropriate for cosmetically treating, providing nutrients to, and/or conditioning the hair, nail, and/or skin via topical application. By "pharmaceutical agent," it is mean any drug that is appropriate for topical use. As used herein, "medicament agents" include those agents capable of promoting recovery from injury and illness.

The cleansing composition may further comprise one or more of the following: water, thickener(s), humectant(s), additional emollient(s), preservative(s), fragrance(s), excipient(s), extract(s), and buffer(s). Various combinations of the foregoing components may be useful in the present invention. The cleansing compositions may be in liquid, cream, balm or gel form, to be applied by hand or a separate applicator, or may be provided in combination with a wipe, sheet or sponge, so as to be applied by an applicator device.

As used herein, "emollients" refer to materials used for cleansing the skin, hair, and eye lashes, the prevention or relief of dryness, or for the protection of the skin. Examples of emollients include, but are not limited to, hydrophobic compounds such as vegetable oils, mineral oils (e.g., petrolatum), fatty esters (e.g., isopropyl palmitate, C12-C15 alkyl benzoate) including those fatty esters of glycerol and the like.

The cleansing composition may comprise the emollient composition in an amount of from about 0.01% to about 60% by weight of the cleansing composition. The cleansing composition may comprise the emollient composition in an amount of from about 1% to about 50% by weight, or from about 2% to about 45% by weight, or from about 5% to about 40% by weight, or from about 10% to about 30% by weight, or from about 15% to about 25% by weight of the cleansing composition, or from about 1.5% to about 24% by weight of the cleansing composition, or from about 2.5% to about 20% by weight of the cleansing composition, or from about 5% to about 12% by weight of the cleansing composition, or from about 7% to about 10% by weight of the cleansing composition. The cleansing composition may comprise the emollient composition in an amount of from about 20% to about 40% by weight of the cleansing composition, or from about 25% to about 35% by weight of the cleansing composition. The emollient composition may be present in the cleansing composition in an amount of about 30% by weight of the cleansing composition.

The cleansing composition may be formed by combining one or more elements concurrently or in any order desired. It is not necessary to separately form an emollient composition from any other components, and the order of addition of components for the cleansing composition may vary as desired. Put another way, when forming the cleansing composition, the additional components may be added with any other components, including each emollient of the emollient composition in any order desired.

The emollient composition comprises three independent and different emollients. The emollient composition may include additional emollients, for example, there may be an additional alkyl ester. Alternatively, or in addition, the emollient composition may include at least one additional ester, which may be an ester of a fatty acid.

The emollient composition therefore includes at least three emollients, where the first emollient, second emollient, and third emollients are each different from each other. The glycol ester of a fatty acid, the alkyl ester of myristate and the alkyl ester of cocoate may be present in the same amount, or substantially the same amount as each other. Alternatively, one of the glycol ester of a fatty acid, the alkyl ester of myristate and the alkyl ester of cocoate may be present in an amount that is greater than the other two emollients. The emollient composition may consist of a glycol ester of a fatty acid, an alkyl ester of myristate, an alkyl ester of cocoate and one or more additional emollients. The emollient composition may consist of a glycol ester of a fatty acid, an alkyl ester of myristate and an alkyl ester of cocoate.

The glycol ester of a fatty acid may comprise a C8 to C22 fatty acid. The glycol ester of a fatty acid may comprise a C8 to C16 fatty acid. The glycol ester of a fatty acid may comprise a C10 to C14 fatty acid. Typically, the glycol ester of a fatty acid comprises a C12 fatty acid. The glycol ester of a fatty acid may be a propylene glycol ester of a fatty acid. The glycol ester of a fatty acid may be propylene glycol laurate. The glycol ester of a fatty acid may consist of propylene glycol laurate. Propylene glycol laurate may be commercially available under the trade name Schercemol PGML.

The alkyl group of the alkyl ester of myristate may be a C3 to C22 alkyl. The alkyl group may be linear, branched or cyclic. The alkyl group may have one, two, three, or four branching points. A branching point may have three bonds to other carbons and one to a hydrogen, or four bonds to other carbons. The side chains attached to a branching point may be methyl, ethyl, propyl, or butyl. Branching points may be at the end of the alkyl group opposite to the ester group or close to the ester group. Non-limiting examples include ethyl-hexyl, isopropyl, isopentyl, neopentyl, isohexyl, isodecyl, isododecyl, and the like. Examples of linear alkyl groups include propyl, hexyl, nonyl, decyl, and hexadecyl. The alkyl group of the alkyl ester of myristate may be a C3 to C12 alkyl, typically a C3 alkyl. The alkyl ester of myristate may be isopropyl myristate. The alkyl ester of myristate may consist of isopropyl myristate. Isopropyl myristate may be commercially available under the trade name Estol 1514.

The alkyl group of the alkyl ester of cocoate may be a C3 to C22 alkyl. The alkyl group of the alkyl ester of cocoate may be a C8 to C16 alkyl. The alkyl group may be linear, branched or cyclic. The alkyl group may have one, two, three, or four branching points. A branching point may have three bonds to other carbons and one to a hydrogen, or four bonds to other carbons. The side chains attached to a branching point may be methyl, ethyl, propyl, or butyl. Branching points may be at the end of the alkyl group opposite to the ester group or close to the ester group. Non-limiting examples include ethyl-hexyl, isopropyl, isopentyl, neopentyl, isohexyl, isodecyl, isododecyl, and the like. Examples of linear alkyl groups include propyl, hexyl, nonyl, decyl, and hexadecyl. The alkyl group of the alkyl ester of cocoate may be a C8 to C14 alkyl, typically a C10 alkyl. The alkyl ester of cocoate may be decyl cocoate. The alkyl ester of cocoate may consist of decyl cocoate. Decyl cocoate may be commercially available by its trade name Tegosoft DC™.

In a preferred emollient composition, the glycol ester of a fatty acid is propylene glycol laurate, the alkyl ester of myristate is isopropyl myristate, and the alkyl ester of cocoate is decyl cocoate.

The emollients of the emollient composition may be synthetic, e.g. derived from petro-oil, or may be derived from renewable resources such as plant and animal material like triglyceride oils, or from fermentation processes, or may be derived from a mixture of resources.

It may be desired to include the first emollient (a glycol ester of a fatty acid), the second emollient (an alkyl ester of myristate) and the third emollient (an alkyl ester of cocoate) in weight amounts with respect to the emollient composition. The first, second, and third emollients may each independently be present in an amount of from about 10% to about 80% by weight of the emollient composition. It may be desired that each of the three emollients be present in approximately the same weight amount with respect to each other (e.g., approximately 33.3% each, by weight of the emollient composition in the case where no additional emollients are present). Alternatively, the first and second emollients may each independently be present in an amount greater than the third emollient, or the first and third emollients may each independently be present in an amount greater than the second emollient, or the second and third emollients may each independently be present in an amount greater than the first emollient. Alternatively, the first, second, or third emollient may be present in an amount greater than the other two emollients.

It may be desired that the first emollient, second emollient, and third emollient be present in a desired ratio with respect to each other. By way of example, the first emollient, the second emollient, and the third emollient may be present in approximately equal amounts by weight (e.g., about 1:1:1 weight ratio). That is, the glycol ester of a fatty acid, the alkyl ester of myristate and the alkyl ester of cocoate may be present in a weight ratio of about 1:1:1.

However, depending upon the desired feel and cleansing desired, it may be preferable to modify the amounts of the three emollients.

The glycol ester of a fatty acid, the alkyl ester of myristate and the alkyl ester of cocoate may be present in a weight ratio of about 1:1:8. The glycol ester of a fatty acid, the alkyl ester of myristate and the alkyl ester of cocoate may be present in a weight ratio of about 1:8:1. The glycol ester of a fatty acid, the alkyl ester of myristate and the alkyl ester of cocoate may be present in a weight ratio of about 8:1:1.

The glycol ester of a fatty acid, the alkyl ester of myristate and the alkyl ester of cocoate may be present in a weight ratio of from about 1:1:1 to about 8:1:1, or from about 1.1:1:1 to about 8:1:1.

The glycol ester of a fatty acid, the alkyl ester of myristate and the alkyl ester of cocoate may be present in a weight ratio of from about 1:1:1 to about 1:8:1, or from about 1:1.1:1 to about 1:8:1.

The glycol ester of a fatty acid, the alkyl ester of myristate and the alkyl ester of cocoate may be present in a weight ratio of from about 1:1:1 to about 1:1:8, or from about 1:1:1.1 to about 1:1:8.

As noted above, the cleansing composition comprises the emollient composition and a surfactant.

The total amount of surfactant present may be from about 0.1% to about 50% by weight of the cleansing composition, or from about 20% to about 50% by weight of the cleansing composition, or from about 30% to about 50% by weight of the cleansing composition, or from about 35% to about 45% by weight of the cleansing composition. The total amount of surfactant present may be about 40% by weight of the cleansing composition. The total amount of surfactant present may be from about 0.1% to about 20% by weight of the cleansing composition, or from about 0.1% to about 10% by weight of the cleansing composition, or from about 0.1% to about 2% by weight of the cleansing composition, or from about 0.5% to about 0.75% by weight of the cleansing composition.

A surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, published in 2001 by Marcel Dekker, Inc., New York, NY) and include, but are not limited to anionic, amphoteric, nonionic and cationic surfactants.

The surfactant comprised in the cleansing composition may comprise a nonionic surfactant, or an anionic surfactant, or an amphoteric surfactant, or a cationic surfactant, or a combination of two or more thereof.

Preferably, the surfactant may comprise a nonionic surfactant. Preferably, the surfactant consists of one or more nonionic surfactants. The nonionic surfactant may include an essentially non-foaming nonionic surfactant. An exemplary suitable essentially non-foaming ionic surfactant includes, for example, a blend of sucrose fatty acid esters. The blend of sucrose fatty acid esters may include sucrose combined with fatty acids such as stearic acid, lauric acid, myristic acid, oleic acid, palmitic acid, and combinations of one or more thereof. One such desirable blend of sucrose fatty acid esters is sucrose cocoate, which is commercially available under the trade name Tegosoft ® LSE, available from Evonik Industries.

By "essentially non-foaming," it is meant that the surfactant has a column height of less than about 20 mm as determined by the Ross-Miles Foam Generation Test. See 18 (I.) Oil & Soap 99-102 (1941) ("Ross-Miles Test"), which is incorporated by reference herein. The cleansing composition and a personal care system comprising the cleansing composition may either be rinseable with water or may be wiped-off. It is desired that any non-foaming nonionic surfactants used in the cleansing compositions described herein be rinseable with water or require no rinsing at all. The non-foaming nonionic surfactant may comprise a blend of sorbitan stearate and sucrose cocoate, available from Croda under the tradename, "Arlacel 2121."

The surfactant may comprise or consist of PEG-7 glyceryl cocoate and hexylene glycol.

If included, the nonionic surfactant may be present in any desired amount, for example, from about 0.1% to about 50% by weight of the cleansing composition, or from about 20% to about 50% by weight of the cleansing composition, or from about 30% to about 50% by weight of the cleansing composition, or from about 35% to about 45% by weight of the cleansing composition. The nonionic surfactant present may be about 40% by weight of the cleansing composition. The nonionic surfactant may be present in an amount of from about 0.1 % to about 20% by weight of the cleansing composition, or from about 0.1% to about 10% by weight of the cleansing composition, or from about 0.1% to about 2% by weight of the cleansing composition, or from about 0.5% to about 0.75% by weight of the cleansing composition.

Alternatively, or in addition, the cleansing composition may comprise an anionic surfactant. As used herein, the term "anionic surfactant" refers to a surfactant having a negatively charged hydrophilic polar group. Examples of anionic surfactants include potassium cetyl phosphate, ammonium lauryl sulfate, sodium laureth sulfate, sodium lauryl sarcosinate, sodium myreth sulfate, sodium pareth sulfate, sodium stearate, sodium lauryl sulfate, $\alpha$ olefin sulfonate, ammonium laureth sulfate, citrates (glyceryl stearate citrate) and fatty acids in their anionic form.

Alternatively, or in addition, the cleansing composition may comprise an amphoteric surfactant. As used herein, the term "amphoteric" shall mean: 1) molecules that contain both acidic and basic sites such as, for example, an amino acid containing both amino (basic) and acid (e.g., carboxylic acid, acidic) functional groups; or 2) zwitterionic molecules which possess both positive and negative charges within the same molecule. The charges of the latter may be either dependent on or independent of the pH of the composition. The amphoteric surfactants are disclosed herein without a counter ion. One skilled in the art would readily recognize that under the pH conditions of the compositions of the present invention, the amphoteric surfactants are either electrically neutral by virtue of having balancing positive and negative charges, or they have counter ions such as alkali metal, alkaline earth, or ammonium counter ions. Examples of amphoteric surfactants suitable for use include, but are not limited to, amphocarboxylates such as alkylamphoacetates (mono or di); alkyl betaines; alkylamidoalkyl betaines (other than CAPB); alkylamidoalkyl sultaines; alkylamphophosphates; phosphorylated imidazolines such as phosphobetaines and pyrophosphobetaines; carboxyalkyl alkyl polyamines; alkylimino-dipropionates; alkylamphoglycinates (mono or di); alkylamphoproprionates (mono or di),); N-alkyl $\beta$-aminoproprionic acids; alkylpolyamino carboxylates; and mixtures thereof.

Alternatively, or in addition, the cleansing composition may comprise a cationic surfactant. Classes of cationic surfactants that are suitable for use include alkyl quaternaries (mono, di, or tri), benzyl quaternaries, ester quaternaries, ethoxylated quaternaries, alkyl amines, and mixtures thereof, wherein the alkyl group has from about 6 carbon atoms to about 30 carbon atoms, with about 8 to about 22 carbon atoms being preferred.

The cleansing composition may further include water. Alternatively, or in addition, the cleansing composition may comprise at least one additional component. The additional component may include, for example, cleansing components such as carriers, thickeners, humectants, glycerides, preservatives, fragrances, and the like.

The cleansing composition may comprise one or more humectants, including aliphatic diols. Other commercially available humectants which are capable of providing moisturization and conditioning properties to the cleansing composition are suitable for use in the present invention. If present, the humectant is preferably present in an amount of from about 0.1 percent to about 4 percent, more preferably from about 0.5 percent to about 2 percent, and most preferably from about 0.5 percent to about 1 percent, based on the overall weight of the cleansing composition. Examples of suitable humectants nonexclusively include: 1) water soluble liquid polyols selected from the group comprising glycerine, propylene glycol, hexylene glycol, butylene glycol, pentylene glycol, dipropylene glycol, and mixtures thereof; 2) polyalkylene glycol of the formula I:

$$HO\text{-}(R''O)_b\text{-}H \qquad (I)$$

wherein R" is an alkylene group having from about 2 to about 4 carbon atoms and b is an integer of from about 1 to about 10, such as PEG 4; 3) polyethylene glycol ether of methyl glucose of formula II:

$$CH_3\text{-}C_6H_{10}O_5\text{-}(OCH_2CH_2)_c\text{-}OH \qquad (II)$$

wherein c is an integer from about 5 to about 25;
4) urea; 5) fructose; 6) glucose; 7) honey; 8) lactic acid; 9) maltose; 10) sodium glucuronate; and 11) mixtures thereof.

In addition to the emollients described above, the cleansing composition may comprise a polymeric emulsifier. Polymeric emulsifiers that may be useful include glycerides or triglycerides, including, for example, caprylic/capric triglyceride or PEG-6 caprylic/capric glyceride. Typically, the polymeric emulsifier may have a molecular weight of at least about 5000. Preferably the polymeric emulsifier is a block copolymer having a hydrophilic portion and a hydrophobic portion. Other examples of suitable polymeric emulsifiers nonexclusively include polyethylene glycol-30 dipolyhydroxystearate available from Croda under the tradename "Cithrol DPHS;" dimethicone copolyol, which is available from Goldschmidt Chemical Corporation under the tradename, "Abil EM® 90"; substituted acrylates such as those available from The Goodrich Corporation under the tradename, "Pemulen®"; and mixtures thereof. When included, the polymeric emulsifier(s) may be present in any desired amount, and may be present in an amount of from about 0.1 % to about 15% by weight of the cleansing composition, or from about 0.1% to about 2% by weight of the cleansing composition, or may be present in an amount of about 0.75% by weight of the cleansing composition.

The cleansing composition may also comprise a preservative or a preservative blend. Suitable preservatives include components such as ethylhexylglycerin, dehydroacetic acid, benzoic acid, phenoxyethanol, polyaminopropyl biguanide, chlorphenesin, PEG-4 Laurate, iodopropynyl butylcarbamate, and mixtures thereof. When included, the preservative may be present in any desired amount, for example, the preservative may be present in an amount of from about 0.01 % to about 2% by weight of the cleansing composition, or may be present in an amount of from about 0.1 to about 1% by weight of the cleansing composition. The preservative blend may include chlorphenesin and phenoxyethanol.

The cleansing composition may also comprise an antioxidant, such as a Vitamin (A, C, E), a plant extract (Coffea arabica, Carotenoid, Prunus cerasus, Olea europaea, Camellia sinensis, Solanum lycopersicum, Lycopene, Vitis vinifera, Resveratrol, Thymus vulgaris, Rosmarinus officinalis), a vegetal fermentation extract (Lactobacillus/Saccharomyces/Turmeric ferment extract filtrate, Panax ginseng cambium cell culture, Saccharomyces/Panax ginseng flower ferment extract, Saccharomyces/Nelumbo nucifera ferment extract filtrate, Monascus/Rice ferment, Saccharomyces/Rice ferment filtrate extract, Rosa damascena callus culture extract, Lactobacillus/Salix alba bark ferment filtrate, Lactobacillus/Spirulina ferment filtrate), or mixture of several vegetal extracts: for example, Saccharomyces/(Lavender/Rosemary/Sage/Thyme) seed extract ferment filtrate.

An antioxidant may be particularly useful in the case of high oil content in the cleansing composition to prevent chemical alteration of the oil, and may also have additional benefit for the skin.

The cleansing composition may also comprise a fragrance or a fragrance blend. Fragrances may include essential oils, or aroma compounds, fixatives, extracts and vitamins and solvents. When included, the fragrance may be present in any desired amount, for example in an amount of from about 0.01% to about 1% by weight of the cleansing composition, or may be present in an amount of about 0.1% to about 0.2% by weight of the cleansing composition.

The emollient composition and any additional components may be formed together to make the cleansing composition, or they may be formed as separate components in the cleansing composition. The designation of an "emollient composition" and "additional component" is not intended to require the separate formation of these two compositions. In processing the cleansing composition, it may be desired to process the emollient composition and any additional components separately, or the emollient composition and any additional components may be processed as a single phase with the order of addition of components being variable.

The cleansing composition may comprise a carrier. In particular, the cleansing composition may comprise water. As described above, the water need not necessarily be processed as a separate and distinct component. Water may be present in the cleansing composition in an amount from about 1% to about 95% by weight of the cleansing composition, or from about 5% to about 50% by weight of the cleansing composition, or from about 20% to about 40% by weight of the cleansing composition, or about 30% by weight of the cleansing composition. Water may be the largest component by weight in the cleansing composition. For example, water may be present in an amount of from about 75% to about 95% by weight of the cleansing composition, or from about 80% to about 85% by weight of the cleansing composition. Alternatively, the cleansing composition may have water in an amount from about 1% to about 30% by weight of the cleansing composition, or from about 2% to about 20% by weight of the cleansing composition, or from about 2.5% to about 5% by weight of the cleansing composition.

The cleansing composition may additionally comprise one or more thickeners, which may be hydrophilic thickeners. Examples of suitable hydrophilic thickeners nonexclusively include carbomers available from B.F. Goodrich under the tradename, "Carbopol ® EDT 2020" (INCI: Acrylates/C10-C30 alkyl acrylate cross polymer), Carbopol® Ultrez 10 NF Polymer (INCI: Carbomer), acrylate copolymers and acrylate crosspolymers, hydroxyethylcellulose modified with cetyl ether groups available from Hercules under the tradename, "Natrosol® Plus", polyvinylmethyl ether/maleic anhydride (PVM/MA) decadiene crosspolymer available from International Specialty Products under the tradename, "Stabileze® QM," and copolymers and mixtures thereof, with carbomers being preferred. Examples of suitable acrylate copolymers nonexclusively include acrylate copolymers available from Rohm & Haas under the tradename, "Aculyn® 33," acrylates/aminoacrylates copolymer available from National Starch & Chemical Company under the tradename, "Structure Plus," acrylates/steareth-20 itaconate copolymer available from National Starch & Chemical Company under the tradename, "Structure 2001," acrylates/ceteth-20 itaconate copolymer available from National Starch & Chemical Company under the tradename, "Structure 3001," acrylates/steareth-20 methacrylate copolymer available from Rohm & Haas under the tradename, "Aculyn ® 22," and copolymers and mixtures thereof. Carbopol EDT 2020 is one component falling under the class of "carbomer interpolymer thickeners", and is useful in the present invention. When included, the thickener may be present in an amount of from about 0.05% to about 0.50% by weight of the cleansing composition, or from about 0.12% to about 0.18% by weight of the cleansing composition.

The cleansing composition may also comprise a buffering agent. Suitable buffering agents may include, for example, citrate buffer, phosphate buffer, lactate buffer, gluconate buffer, and sodium hydroxide. The buffering agent or agents may be present in any amount desirable to achieve the desired pH. The buffering agent may be a blend of 20% sodium hydroxide and water or other solvent. When included, the buffering agent may be present in an amount of about 0.01% to about 0.5% by weight of the cleansing composition, and more desirably about 0.2% by weight of the cleansing composition.

The emollient composition may be formed separately from other additional components, or may include the emollient composition formed with other additional components except for a carrier, e.g. water, thereby forming a concentrated composition. The concentrated composition may be added to the carrier, e.g. water, separately.

The cleansing composition may comprise, for example, about 30% of the emollient composition, about 40% of surfactant(s) and about 30% of water by weight of the cleansing composition.

The cleansing and emollient compositions described herein are useful in removing various cosmetic (that is, makeup) products from the skin of users, and in particular, are useful in removing mascara, foundation, lipstick and eyeliner efficiently and without a remaining heavy or greasy residue. Mascara is a notoriously difficult cosmetic material to remove from skin, as it deposits high levels of film formers and includes a relatively high level of hydrophobic materials. Further, mascara includes a high level of carbon dark pigments, giving it a dark color. In addition, since mascara is typically applied on the eyes or eyelashes, cleansing requires a gentle, low pressure and non-planar application. Eyeliner presents its own difficulties in removal, as it includes titanium dioxide, iron oxide, mica, silica and higher concentration of pigments/colorants, which may be more difficult to remove. In addition, as with mascara, eyeliner is often applied to the eyes or eye region, and cleansing requires a gentle, lower pressure and non-planar application. Foundation provides some difficulty as well, as it is typically an emulsion and may include silicones, which cause it to remain on the skin. Cosmetics today are geared towards a "24-hour" use, where the cosmetic remains on the skin of the user for an extended period of time. These "long-lasting" and waterproof cosmetics have a high transfer resistance, making them difficult to remove.

To provide a material that effectively and sufficiently removes enough of these three cosmetic materials (mascara, foundation and lipstick) is particularly desired. Further, it is desired to provide a composition that does not leave an oily or greasy residue on the surface of the skin, since such oily cleansers may be difficult to remove and leave the user with an unclean feeling. In addition, it is useful that the cleansing composition provides low or no irritation to the skin, particularly the eyes. A soft and pleasant feel to the user's skin during and after use is also desired. The present invention provides an emollient composition and a cleansing composition comprising said emollient composition that has desirable characteristics defined herein, while also providing suitable cleansing capabilities.

The cleansing composition may be used as a liquid or gel composition, applied to the skin by the user's hands or other tool. Optionally, the cleansing composition may be soaked into or embedded into a substrate, such as a wipe or other application tool, where the user may simply remove the wipe or other application tool from a package and use the wipe or other application tool to achieve cleansing. The cleansing composition may be soaked into an individual wipe, and the user may use that wipe to cleanse the user's skin. Individual wipes may be packaged in a dispenser that includes a plurality of wipes, or may be packaged in a dispenser that includes only one wipe. Desirable substrates include wipes described in greater detail below.

The cleansing composition may further optionally contain one or more benefit agents or pharmaceutically-acceptable salts thereof. As used herein, the term "benefit agent" includes any active ingredient that is to be delivered into and/or onto the skin, hair or nail at a desired location, such as a cosmetic agent or a pharmaceutical agent. By "cosmetic agent," it is meant any ingredient that is appropriate for cosmetically treating, providing nutrients to, and/or conditioning the hair, nail, and/or skin via topical application. By "pharmaceutical agent," it is mean any drug that is either hydrophobic or hydrophilic in nature and appropriate for topical use. As used herein "medicament agents" include those agents capable of promoting recovery from injury and illness.

The benefit agents useful herein may be categorized by their therapeutic benefit or their postulated mode of action. However, it is to be understood that the benefit agents useful herein may, in some circumstances, provide more than one therapeutic benefit or operate via greater than one mode of action. Therefore, the particular classifications provided herein are made for the sake of convenience and are not intended to limit the benefit agents to the particular application(s) listed. In addition, the compounds, which are identified below as being suitable for use as benefit agents, may be used in an amount over and above the amount that they may be used for other purposes in the cleansing composition.

Examples of suitable benefit agents include, but are not limited to, depigmentation agents; reflectants; film forming polymers; humectants; amino acids and their derivatives; antimicrobial agents; allergy inhibitors; anti-acne agents; anti-aging agents; anti-wrinkling agents, antiseptics; analgesics; antitussives; antipruritics; local anesthetics; anti-hair loss agents; hair growth promoting agents; hair growth inhibitor agents, antihistamines such as Mandragora Vernalis, Tanacetum Parthenium and the like; antiinfectives such as Acacia Catechu, Aloe Barbadensis, Convallaria Majalis, Echinacea, Eucalyptus, Mentha Piperita, Rosa Canina, Sassafras Albidum, and the like; inflammation inhibitors; anti-emetics; anticholinergics; vasoconstrictors; vasodilators; wound healing promoters; peptides, polypeptides and proteins; deodorants and antiperspirants; medicament agents; skin emollients and skin moisturizers; skin firming agents, vitamins; tanning agents; skin lightening agents; antifungals such as Centaurea Cyanus, Kalmia Latifolia and antifungals for foot preparations; depilating agents; shaving preparations; external analgesics; perfumes; counterirritants; hemorrhoidals; insecticides; poison ivy products; poison oak products; burn products; anti-diaper rash agents; prickly heat agents; vitamins; amino acids and their derivatives; herbal extracts; retinoids; flavenoids; sensates; anti-oxidants; skin conditioners; hair lighteners; chelating agents; cell turnover enhancers; coloring agents; sunscreens, those active ingredients disclosed in U.S. Pat. No. 6,063,397, which is incorporated herein by reference, anti-edema agents, collagen enhancers, and mixtures thereof.

Examples of suitable anti-edema agents nonexclusively include bisabolol natural, synthetic bisabolol, and mixtures thereof.

Examples of suitable vasoconstrictors nonexclusively include horse chestnut extract, prickly ash, and mixtures thereof.

Examples of suitable anti-inflammatory agents nonexclusively include benoxaprofen, centella asiatica, bisabolol, feverfew (whole), feverfew (parthenolide free), green tea extract, green tea concentrate, hydrogen peroxide, lycopene including "Lyc-o-Pen" available from LycoRed Natural Products Industries, Ltd., oat oil, chamomile, and mixtures thereof.

Examples of collagen enhancers nonexclusively include vitamin A, vitamin C, and mixtures thereof.

Examples of suitable skin firming agent nonexclusively include dimethylaminoethanol ("DMAE").

Examples of suitable antipruritics and skin protectants nonexclusively include oatmeal, betaglucan, feverfew, soy and derivatives thereof, bicarbonate of soda, colloidal oatmeal, surfactant based colloidal oatmeal cleanser, Anagallis Arvensis, Oenothera Biennis, Verbena Officinalis, and the like. These antipruritics may be used in an amount, based upon the total weight of the cleansing composition, from about 0.01 percent to about 40 percent, and preferably from about 1 percent to about 5 percent.

As used herein, colloidal oatmeal means the powder resulting from the grinding and further processing of whole oat grain meeting United States Standards for Number 1 or Number 2 oats. The colloidal oatmeal has a particle size distribution as follows: not more than 3 percent of the total particles exceed 150 micrometers in size and not more than 20 percent of the total particles exceed 75 micrometers in size. Examples of suitable colloidal oatmeals include, but are not limited to, "Tech-O" available from the Beacon Corporation and colloidal oatmeals available from Quaker.

Examples of suitable reflectants nonexclusively include mica, alumina, calcium silicate, glycol dioleate, glycol distearate, silica, sodium magnesium fluorosilicate, and mixtures thereof.

Suitable film forming acetyl tyrosinamide, zinc pyrithione, coal tar, benzoyl peroxide, selenium sulfide, hydrocortisone, sulfur, menthol, pramoxine hydrochloride, tricetylmonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolyzed wheat proteins, hydrolyzed silk proteins, octyl methoxycinnamate, oxybenzone, minoxidil, titanium dioxide, zinc dioxide, retinol, erthromycin, tretinoin, and mixtures thereof.

One type of benefit agent includes those therapeutic components that are effective in the treatment of dandruff, seborrheic dermatitis, and psoriasis as well as the symptoms associated therewith. Examples of such suitable benefits agents nonexclusively include zinc pyrithione, anthralin, shale oil and derivatives thereof such as sulfonated shale oil, selenium sulfide, sulfur; salicylic acid; coal tar; povidone-iodine, imidazoles such as ketoconazole, dichlorophenyl imidazolodioxalan, which is commercially available from Janssen Pharmaceutica, N.V., under the tradename, "Elubiol", clotrimazole, itraconazole, miconazole, climbazole, tioconazole, sulconazole, butoconazole, fluconazole, miconazole nitrate and any possible stereo isomers and derivatives thereof; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, e.g. vitamin A palmitate, retinoids, retinols, and retinoic acid; corticosteroids such as hydrocortisone, clobetasone, butyrate, clobetasol propionate and mixtures thereof.

The amount of benefit agent to be combined with the cleansing composition or the emollient composition may vary depending upon, for example, the ability of the benefit agent to penetrate through the skin, hair or nail, the specific benefit agent chosen, the particular benefit desired, the sensitivity of the user to the benefit agent, the health condition, age, and skin, hair, and/or nail condition of the user, and the like. The benefit agent is used in a "safe and effective amount," which is an amount that is high enough to deliver a desired skin, hair or nail benefit or to modify a certain condition to be treated, but is low enough to avoid serious side effects, at a reasonable risk to benefit ratio within the scope of sound medical judgment. If included, a benefit agent may be present in the cleansing composition or personal care system in an amount, based upon the total weight of the composition/system, from about 0.01 percent to about 5.0 percent, and preferably from about 0.01 percent to about 2.0 percent, and more preferably from about 0.01 percent to about 1.0 percent.

Optionally, commercially available detergent thickeners that are capable of imparting the appropriate viscosity to conditioning compositions are suitable for use in this invention. If used, the detergent thickeners should be present in the compositions in an amount sufficient to raise the Brookfield viscosity of the composition to a value of between about 500 to about 10,000 centipoise. Examples of suitable detergent thickeners nonexclusively include: mono or diesters of polyethylene glycol of formula III.

$$HO\text{-}(CH_2CH_2O)_zH \qquad (III)$$

wherein z is an integer from about 3 to about 200;

fatty acids containing from about 16 to about 22 carbon atoms; fatty acid esters of ethoxylated polyols; ethoxylated derivatives of mono and diesters of fatty acids and glycerine; hydroxyalkyl cellulose; alkyl cellulose; hydroxyalkyl alkyl cellulose; and mixtures thereof. More specifically, suitable detergent thickeners nonexclusively include behenalkonium chloride; cetyl alcohol, quaternium-46, hydroxyethyl cellulose, cocodimonium chloride, polyquaternium-6, polyquaternium-7, quaternium-18, PEG-18 glycerol oleate/cocoate, a mixture of acrylates/steareth-50 acrylate copolymer, laureth-3 and propylene glycol, which is commercially available from Goldschmidt under the tradename "Antil 208," a mixture of cocamidopropylbetaine and glyceryl laurate which is commercially available from Goldschmidt under the tradename, "Antil HS60," a mixture of propylene glycol, PEG 55, and propylene glycol oleate, which is commercially available from Goldschmidt under the tradename, "Antil 414 liquid," and mixtures thereof. Preferred detergent thickeners include polyethylene glycol ester, and more preferably PEG-150 distearate which is available from the Stepan Company of Northfield, Ill. Or from Comiel, S.p.A. of Bologna, Italy under the tradename, "PEG 6000 DS".

The above described cleansing composition may be prepared by combining the desired components in a suitable container and mixing them under any desired conditions in any conventional mixing means well known in the art, such as a mechanically stirred propeller, paddle, and the like. Processing may include the separate steps of forming the emollient composition and adding to the emollient composition one or more additional components to produce the cleansing composition. Alternatively, the cleansing composition may be formed by combining the components in any desired order, where the order of addition of components is variable. Alternatively, the cleansing composition may be formed by combining the components in any order with the exception of water, and then separately adding water to the premix to form the cleansing composition.

The cleansing composition may be provided in a container in liquid, gel, balm or cream form, whereby it may be applied to the skin by a user by hand or a cloth, wipe, sheet, or other device, or alternatively the cleansing composition may be embedded or soaked into one wipe or a plurality of sheets or wipes, whereby the cleansing composition may be applied to the skin by wiping the sheet, wipe, or other device.

Since the cleansing composition is to be applied to the face and is desirably used to remove cosmetic materials, it may be desired that the composition be soaked into a sheet or a wipe and provided to the user in this form. The user then removes the soaked wipe from a suitable air-tight package, and applies it directly to the skin. Thus, a system or package may include a plurality of wipes in a resealable package, where each wipe has been soaked in or otherwise contains a cleansing composition of the present invention. In other aspects, one individual wipe may be contained in its own air-tight package or container, where the package may be discarded after use of the wipe contained therein. The wipes are desirably disposable and include degradable components, rendering them environmentally friendly and sound. Wipe materials can include, for example, natural, biodegradable or synthetic fibers or filaments (some examples of which include: wool, silk, jute, hemp, cotton, linen, sisal, or ramie) or synthetic (some examples of which include: rayon, cellulose ester, polyvinyl derivatives, polyolefins, such as polyethylene and polypropylene, polyamides, such as nylon 6, nylon 6,6, or polyesters, such as polyethylene terephthalate and polybutylene terephthalate), or combinations thereof. These nonwoven materials are generally described in the INDA "NONWOVEN FABRICS HANDBOOK", (1999), for nonwoven substrates and their methods of manufacture. One particular substrate that may be useful is an embossed spunlace non-woven material made from a mixture of 20% Rayon 1.7 dtex, 40% Polyester (PET) 1.3 dtex and 40% Polyester (PET) 1.7 dtex. The basis weight of the substrate may vary, but generally ranges from about 20 grams per square meter to about 500 grams per square meter, for example from about 50 grams per square meter to about 150 grams per square meter. Wipe substrates may be any desired size, and may have any desired area, including from about 2-10 inches in length and about 2-10 inches in width, more desirably about 7-8 inches in length and about 7-8 inches in width. Other suitable wipe materials include those described in EP 1 283 019 B1, U.S. Patent No. 9,622,944 and US Patent Application No. 2016/0367102, the entire disclosures of each of which are incorporated by reference herein in their entireties.

The composition may be provided to a user in a liquid, balm, or gel dispensing bottle or container, where the user applies the composition by the hand, or applies the composition onto a wipe or other applicator device, and the user uses that composition-applied applicator to cleanse the skin. The composition may be sold in a dispensing bottle by itself or may be sold in a kit wherein the package includes a dispensing bottle with the composition therein and a plurality of applicator devices, such as wipes or balls.

The present invention includes not only the cleansing composition described above, but also includes a method of using the cleansing composition described above. The method includes applying an effective amount of the composition to the skin, including facial skin and near ocular skin, and cleansing the skin with the composition. The composition may be applied to the skin by hand or through use of an applicator, such as a sheet, wet wipe, sponge, brush, and the like. The composition may be applied while the skin is wet or dry, and may be wiped off the skin, rinsed off the skin, or the skin may be allowed to dry after application of the composition. It is particularly desired that the composition be applied to the skin via a pre-soaked wipe and is allowed to dry off the skin without further washing or cleaning by the user. The used wipe may be disposed of by the user. The invention described herein may be practiced in the absence of any component, ingredient or step not specifically disclosed herein or may include additional components or steps that are not expressly disclosed herein.

As noted above, the present invention is directed to compositions that can be used to clean a variety of cosmetics off the surface of a user's skin effectively and efficiently. The inventive compositions should be capable of removing, for example, foundation, lipstick, eyeshadow, lipliner, eyebrow, pencil, pomade, blush mascara and eyeliner efficiently. Further, the inventive compositions may be substantially free of silicones, if not entirely free of silicones.

The present invention may be better understood through the following examples, which are exemplary in nature and not intended to be limiting to any specific combination of elements.

Examples

The following Examples describe the formation of compositions that may be useful either by themselves or as part of a cleansing composition to cleanse various makeup materials from the skin of users. The method used to assess the cleansing efficiency of makeup removers is a combination of a mechanical cleaning with a texture analyzer, a controlled light image capture system and an image analysis system with Matlab.

The texture analyzer used in these Examples is called a texturometer, which provides a reproducible mechanical movement to clean dirty model surfaces with makeup remover products. The texture analyzer considers force, distance and time simultaneously. The speed can be fixed and the variations of forces may then be measured, or the displacement can be controlled in order to maintain a constant force. To do so, this equipment has a force captor.

The texturometer used in these Examples is called TA.XT-plus, from Stable Micro Systems, UK. TA.XT-plus was used for all the measurements described herein. The software used was Texture Exponent32, from Stable Micro Systems, Cardiff, UK.

For friction measurements, the following was used: a friction rig HFS (Horizontal Friction System, *Stable Micro Systems*) and a mobile of 204 g weight and 15x8 cm dimension, fixed with a screw to the texturometer were used.

Sample plates of PMMA (PolyMethylMethAcrylate) (80 × 80 cm) were used to mimic skin support. It is possible to use other materials like Bioskin plate (Beaulax Co ltd, Saitama, Japan), ABS (acrylonitrile butadiene styrene), PVC, HDPE, if desired.

To fix the sample plates, a solid support was made in 3D printing (PLA, Polylactic acid). The solid support was fixed on the tray of the friction rig and taped so that it does not move when the mobile is going back and forth on top of it.

A Canon camera with its lens (Nikon D90 (Nikon Corp Japan) + Nikon AF MICRO NIKKOR 105mm1:2.8 D) was used for the analysis of each sample described herein. It was positioned so that it can photograph the cleaning tests. It was held in place with a camera arm (angle 145°). A photo studio (80 x 80 x 80 cm) containing 2 LED was used to optimize the quality of pictures. The texturometer and camera were placed inside.

For the tests, the protocol of the makeup plates preparation was as follows:

◦ Fix the film applicator (100mm BEVS 1806B/100 BEVS INDUSTRIAL CO. LIMITED, Guangzhou, China), with double-sided adhesive tape, on a laboratory bench;

◦ Weigh a PMMA plate;

◦ On the PMMA plate, stick a template paper with a circular hole (diameter 2.5cm) in the middle;

◦ Fill the hole with makeup;

◦ Adjust the height of the film applicator according to the makeup (about 2.6mm for mascara and lipstick, about 2.45mm for foundation but this height can slightly change according to the thickness of the PMMA plate) (Thickness = PMMA plate + tested makeup);

◦ Pass the PMMA plate below the film applicator;

◦ Remove the template paper and weigh the plate, and therefore determine the makeup mass on the plate,

◦ Let the plate dry for 12 hours to 48 hours maximum, at room temperature (approximately 20 degrees C).

For mobile calibration, it was desired to calibrate the probe's height with the mobile as close to the friction rig as possible. The "removable support" is the friction rig HFS (Horizontal Friction System, *Stable Micro Systems*) and the mobile of 204 g weight and 15x8 cm dimension.

To measure the results, the following setup process was used: A cotton pad (Demak up®, cotton science™ expert honeycomb texture 100% biodegradable natural cotton fibres; from Essity SA, Belgium) was fixed with double-sided adhesive tape on the removable support. Then, 3 mL of the emollient (or emollient composition) was put on the cotton pad, the removable support was flipped on the fixed support and a mass of 2 kg is placed on the removable support.

The analysis for makeup remover efficiency was performed as follows: The removable support was configured to be automatically displaced by 70mm. Displacement was reproduced several times based on the type of makeup investigated: 5 times for mascara and lipstick, 2 times for foundation. Pictures of the PMMA plate were taken before any movement (the "before image") and at the end of removable support displacements sequence (the "after image"). The pictures thus obtained for each sample (before / after) were analyzed with Matlab to assess the cleansing efficiency of the emollients.

For mascara, the colors were evaluated in the before image and the after image, and each was given a mean value of the color of each pixel in the circle where the makeup was applied in the "before image" and the "after image" (the mean value referred to as Bval). The Bval for the "after image" was compared to the Bval for the "before image", and the ratio given a Bratio number (Bval for "after image" / Bval for "before image"). The Bratio was determined to be more relevant for mascara, since it assesses grey shades effectively.

For lipstick and foundation, which include more colored hues than mascara, a clean plate test was determined to be more relevant as an evaluator of cleansing ability. For each test, the clean plate value was defined as ((number of elements in the chroma matrix - number of non zero elements in the chroma matrix)/number of elements in the chroma matrix)*100.

The Chroma matrix in the Lab color space is: $C^*_{ab} = \sqrt{a^{*2} + b^{*2}}$    a* (green to red) and b* (blue to yellow) are respectively the 2nd and 3rd dimension of the image in the lab color space.

The makeup products tested included the following:

◦ Mascara: Neutrogena hydroboost plumping mascara waterproof

◦ Lipstick: L'Oréal color rich n°297 Red passion

◦ Foundation: L'Oréal accord parfait 3D beige doré

The various emollients tested in the following examples included:

◦ Schercemol PGML ; INCI name : Propylene Glycol Laurate ; supplier: Lubrizol

◦ Schercemol IDO; INCI name : Isodecyl Oleate ; supplier: Lubrizol

◦ Estol 1514; INCI name : Isopropyl Myristate ; supplier Croda

◦ Cegesoft PFO ; INCI name : Passiflora Incarnata Oil ; supplier BASF

◦ Crodamar 227 ; INCI name : Decyl isooctadecanoate; supplier Croda

◦ Tegosoft DC MB ; INCI name : Decyl Cocoate ; supplier Evonik

◦ Arlamol HD ; INCI name : Isohexadecane; supplier Croda

◦ Apricot kernel organic cold pressed virgin oil; INCI name : Prunus armeniaca kernel oil ; supplier GreenTech

Individual Emollients

In a first set of experiments, the cleansing properties of a selected group of emollients were investigated. The emollients were tested alone, in the form obtained from the suppliers (i.e., not in combination with other emollients or in a formulation).

Bval and Bratio measurements were obtained for mascara, and clean plate measurement were obtained for lipstick and foundation, as described above.

1. Mascara

Bval and Bratio values for the selected emollients on a mascara stain are presented in Table 1 below. For mascara, the Bratio value is considered a relevant indicator of the ability to remove mascara from the surface, with higher Bratio values indicating a better cleansing efficacy score.

Table 1. Bratio and Bval for Mascara Removal

|  | Bratio Average | Bratio Standard Deviation | Bval Average | Bval Standard Deviation |
|---|---|---|---|---|
| Estol 1514 | 2,46 | 0,19 | 103,8 | 9,1 |
| Cegesoft PFO | 0,95 | 0,03 | 107,1 | 13,4 |
| Arlamol HD | 2,42 | 0,09 | 107,8 | 7,4 |
| Schercemol IDO | 2,09 | 0,27 | 107,9 | 3,7 |
| Crodamar 227 | 2,05 | 0,02 | 108,4 | 1,8 |
| Tegosoft DC MB | 2,32 | 0,04 | 109,1 | 4,1 |
| Schercemol PGML | 1,75 | 0,11 | 113,0 | 2,2 |
| Prunus armeniaca | 1,46 | 0,09 | 113,4 | 2,0 |

As can be seen from Table 1, Estol1514 was found to have the highest Bratio value with regard to removal of mascara stain. Other relevant emollients identified in the Table above include Arlamol HD, Tegosoft DC MB and Schercemol IDO.

2. Lipstick

Clean plate values for the selected emollients on a lipstick stain are presented in Table 2 below. Higher clean plate values are deemed to be related to a better cleansing profile.

Table 2. Clean Plate Value - Lipstick

| clean plate | Lipstick | |
|---|---|---|
|  | Average | Standard deviation |
| Crodamar 227 | 69,27 | 27,41 |
| Schercemol IDO | 51,60 | 20,22 |
| Prunus armeniaca | 50,98 | 36,67 |
| Estol 1514 | 49,74 | 15,27 |
| Arlamol HD | 48,99 | 14,74 |
| Tegosoft DC MB | 43,77 | 5,01 |
| Schercemol PGML | 36,82 | 3,78 |
| Cegesoft PFO | 36,48 | 18,33 |

As can be seen from Table 2, Crodamar227 was identified as the most promising emollient to remove lipstick stain. Other relevant emollients such as Schercemol IDO, Prunus armeniaca, Estol1514 and Arlamol HD show comparable results.

3. Foundation

Clean plate values for the selected emollients on a foundation stain are presented in Table 3 below. Higher clean plate values are deemed to be related to a better cleansing profile.

Table 3. Clean Plate Value - Foundation

| clean plate | Foundation | |
|---|---|---|
| | Average | Standard deviation |
| Estol 1514 | 99,34 | 0,64 |
| Arlamol HD | 98,28 | 1,23 |
| Schercemol PGML | 96,45 | 2,45 |
| Tegosoft DC MB | 94,56 | 5,95 |
| Schercemol IDO | 94,34 | 0,00 |
| Crodamar 227 | 81,84 | 5,29 |
| Prunus armeniaca | 81,83 | 28,39 |
| Cegesoft PFO | 56,70 | 40,50 |

As can be seen from Table 3; Esto11514, Arlamol HD, Schercemol PGML, Tegosoft DC MB and Schercemol IDO all showed promising foundation stain cleansing properties.

Combinations of Emollients

In a second set of experiments, the cleansing properties of emollient compositions comprising combinations of emollients were investigated to see if a combination showing promising results on all three type of makeup stains (mascara, lipstick and foundation) could be identified. The emollients proportions are indicated in weight % in the tested emollient compositions.

A number of combinations were tested, and the data presented here is focused on the most promising results comprising a mixture of Tegosoft DC MB, Estol1514 and Schercemol PGML.

1. Mascara

Bratio values for emollient compositions comprising different proportions of the three selected emollients on mascara stains are presented in Table 4.

Table 4. Combination of Emollients - Mascara

| Emollients | Amount (weight ratio) Tegosoft: Estol : Schercemol | Bratio Average | Standard deviation |
|---|---|---|---|
| Tegosoft DC MB Estol 1514 Schercemol PGML | 1:1:1 | 2,93 | 0,38 |
| Tegosoft DC MB Estol 1514 Schercemol PGML | 1:1:8 | 2,92 | 0,46 |
| Tegosoft DC MB Estol 1514 Schercemol PGML | 8:1:1 | 2,30 | 0,00 |
| Tegosoft DC MB Estol 1514 Schercemol PGML | 1:8:1 | 2,29 | 0,24 |

Bratio values as high as 2,93 were obtained for the mixture of the 3 selected emollients. In comparison, with reference to the Table above, Bratio values of the 3 emollients taken separately were 2,46 (Estol1514), 2,32 (Tegosoft DC MB) and 1,75 (Schercemol PGML). Cleansing efficacy over mascara stain was therefore determined to be slightly improved compared to the individual emollients. In particular, when the three emollients were in approximately equal amounts, or when a higher amount of Schercemol PGML was used, improved cleansing efficacy was seen.

2. Lipstick

Clean plate values for mixtures comprising different proportions of the three selected emollients on lipstick stains are presented in Table 5.

Table 5. Combination of Emollients - Lipstick

| | Amount (weight ratio) Tegosoft: Estol : Schercemol | Clean Plate Average | Standard deviation |
|---|---|---|---|
| Tegosoft DC MB Estol 1514 Schercemol PGML | 1:8:1 | 91,88 | 0,92 |
| Tegosoft DC MB Estol 1514 Schercemol PGML | 8:1:1 | 77,02 | 7,32 |
| Tegosoft DC MB Estol 1514 Schercemol PGML | 1:1:8 | 66,19 | 9,89 |
| Tegosoft DC MB Estol 1514 Schercemol PGML | 1:1:1 | 63,20 | 0,32 |

Clean plate values as high as 91,88 were obtained for the mixture of the 3 selected emollients. In comparison, and with reference to the Table above, clean plate values of the 3 emollients taken separately were 49,74 (Estol1514), 43,77 (Tegosoft DC MB) and 36,82 (Schercemol PGML). Cleansing efficacy over lipstick stain was seen to be greatly improved compared to the individual emollients. Cleansing efficacy of the mixture in each ratio was higher than the individual efficacy of each of these emollients. Further, the combination when Estol 1514 was in a higher amount or when Tegosoft DC MB was in a higher amount was found to be superior to the most effective emollient identified in the first set of experiment: Crodamar227 (69,27). Even when Schercemol PGML was in the highest amount, or when the amounts were approximately equal, the cleansing efficacy was similar to the individual Crodamar227.
Surprisingly the cleansing efficacy of a moderately effective emollient (Estol1514) was boosted by 2 other emollients showing amongst the lowest efficacies.

3. Foundation

Clean plate values for mixtures comprising different proportions of the 3 selected emollients on foundation stains are presented in Table 7.

Table 6. Combination of Emollients - Foundation

| | Amount (weight ratio) Tegosoft: Estol : Schercemol | Clean Plate Average | Standard deviation |
|---|---|---|---|
| Tegosoft DC MB Estol 1514 Schercemol PGML | 1:1:1 | 94,83 | 0,13 |
| Tegosoft DC MB Estol 1514 Schercemol PGML | 1:1:8 | 94,06 | 2,40 |
| Tegosoft DC MB Estol 1514 Schercemol PGML | 1:8:1 | 87,40 | 3,77 |
| Tegosoft DC MB Estol 1514 Schercemol PGML | 8:1:1 | 77,74 | 12,54 |

Clean plate values of up to 94,8 were obtained for the mixture of the 3 selected emollients, when the three were in approximately equal amounts. Clean plate values of the 3 emollients taken separately were similar ranging from 99,3 (Estol1514) to 94,5 (Tegosoft DC MB), as seen above. The cleansing efficacy of the individual emollients was fairly high for foundation, and thus the cleansing efficacy of the blended combination regarding removal of the foundation stain is comparable to the emollients taken separately.

Conclusion

Based upon the individual cleansing tests set forth above, and the cleansing tests of the three emollients blended together, it can be seen that a combination of Tegosoft DC MB, Estol1514 and Schercemol PGML showed superior cleansing results for the removal of makeup stains including mascara, lipstick and foundation. Results for the blended combination of emollients to remove lipstick and mascara were improved compared to the emollients taken separately. Cleansing results for the blended combination to remove foundation were aligned with the observations made on the emollients taken separately.

In particular, the cleansing results for the blended combination of emollients to remove lipstick were surprisingly high as compared to the results of the emollients taken separately. Therefore, the combination of the three emollients was determined to be more effective at removing makeup components overall.

**Claims**

1. An emollient composition comprising:

   A. a glycol ester of a fatty acid;
   B. an alkyl ester of myristate; and
   C. an alkyl ester of cocoate.

2. The emollient composition according to claim 1, wherein the glycol ester of a fatty acid comprises a C8 to C22 fatty acid.

3. The emollient composition according to claim 1 or claim 2, wherein the glycol ester of a fatty acid is a propylene glycol ester of a fatty acid.

4. The emollient composition according to claim 3, wherein the glycol ester of a fatty acid is propylene glycol laurate.

5. The emollient composition according to any preceding claim, wherein the alkyl group of the alkyl ester of myristate is a C3 to C22 alkyl.

6. The emollient composition according to any preceding claim, wherein the alkyl ester of myristate is isopropyl myristate.

7. The emollient composition according to any preceding claim, wherein the alkyl group of the alkyl ester of cocoate is a C3 to C22 alkyl.

8. The emollient composition according to claim 7, wherein the alkyl ester of cocoate is decyl cocoate.

9. The emollient composition according to any preceding claim, wherein the glycol ester of a fatty acid is propylene glycol laurate, the alkyl ester of myristate is isopropyl myristate, and the alkyl ester of cocoate is decyl cocoate.

10. The emollient composition according to any preceding claim, wherein the glycol ester of a fatty acid, the alkyl ester of myristate and the alkyl ester of cocoate are present in a weight ratio of about 1:1:1.

11. The emollient composition according to any of claims 1 to 9, wherein the glycol ester of a fatty acid, the alkyl ester of myristate and the alkyl ester of cocoate are present in a weight ratio of from about 1.1:1:1 to about 8:1:1.

12. The emollient composition according to any of claims 1 to 9, wherein the glycol ester of a fatty acid, the alkyl ester of myristate and the alkyl ester of cocoate are present in a weight ratio of from about 1:1.1:1 to about 1:8:1.

13. The emollient composition according to any of claims 1 to 9, wherein the glycol ester of a fatty acid, the alkyl ester of myristate and the alkyl ester of cocoate are present in a weight ratio of from about 1:1:1.1 to about 1:1:8.

14. The emollient composition according to any of claims 1 to 9, wherein the glycol ester of a fatty acid is present in an amount of from about 5% to about 20% by weight of the emollient composition.

15. A cleansing composition comprising the emollient composition of any of the preceding claims and a surfactant.

16. The cleansing composition of claim 15, further comprising water, wherein the water is present in an amount of from about 1% to about 95% by weight of the cleansing composition.

17. The cleansing composition of claim 16, wherein the water is present in an amount of from about 5 to about 50% by weight of the cleansing composition, or from about 20% to about 40% by weight of the cleansing composition.

18. The cleansing composition of any of claims 15 to 17, wherein the total amount of surfactant present is from about 30% to about 50% by weight of the cleansing composition.

19. The cleansing composition of any of claims 15 to 18, wherein the surfactant comprises PEG-7 glyceryl cocoate and hexylene glycol.

20. The use of a cleansing composition of any of claims 15 to 19 to remove at least one of mascara, lipstick, and foundation from the surface of skin.

Europäisches
Patentamt

European
Patent Office

Office européen
des brevets

# EUROPEAN SEARCH REPORT

Application Number

EP 22 30 6243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 848 507 B1 (MERCK PATENT GMBH [DE]; SACHTLEBEN CHEMIE GMBH [DE]) 5 August 2009 (2009-08-05) | 1,2,5-8, 13,15,16 | INV. A61K8/37 A61Q1/14 |
| Y | * page 33, paragraph 0202; example Sonnenschutzlotion O/W * | 1-8, 10-19 | A61Q19/10 |
| A |  | 9 |  |
|  | ----- |  |  |
| X | DATABASE GNPD [Online] MINTEL; 20 January 2020 (2020-01-20), anonymous: "Protective Eye Cream", XP93019807, Database accession no. 7180773 * abstract * | 1,2,5-7, 15 |  |
|  | ----- |  |  |
| Y | DATABASE GNPD [Online] MINTEL; 23 April 2007 (2007-04-23), anonymous: "Eye & Lip Makeup Remover", XP055901284, Database accession no. 695185 | 1-8, 10-20 |  |
| A | * abstract * | 9 |  |
|  | ----- |  | TECHNICAL FIELDS SEARCHED (IPC) |
| Y | DATABASE GNPD [Online] MINTEL; 30 January 2008 (2008-01-30), anonymous: "Make-Up Remover Deep Cleansing Milk", XP93019809, Database accession no. 852619 | 1-8, 10-20 | A61K A61Q |
| A | * abstract * | 9 |  |
|  | ----- |  |  |
| Y | US 2010/137179 A1 (RUSSELL MICHAEL [US] ET AL) 3 June 2010 (2010-06-03) | 1-8, 10-20 |  |
| A | * claim 1; tables II, III * | 9 |  |
|  | ----- |  |  |
| Y | US 2022/023160 A1 (KARIYA TOMOYUKI [JP]) 27 January 2022 (2022-01-27) | 1-8, 10-19 |  |
| A | * claims 1,6 * | 9 |  |
|  | ----- |  |  |

The present search report has been drawn up for all claims

| Place of search | Date of completion of the search | Examiner |
|---|---|---|
| Munich | 3 February 2023 | Grenouillat, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 30 6243

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2023

| Patent document cited in search report | | Publication date | Patent family member(s) | | Publication date |
|---|---|---|---|---|---|
| EP 1848507 | B1 | 05-08-2009 | AT | 438445 T | 15-08-2009 |
| | | | DE 102005007482 | A1 | 14-09-2006 |
| | | | EP | 1848507 A1 | 31-10-2007 |
| | | | ES | 2329825 T3 | 01-12-2009 |
| | | | WO | 2006087066 A1 | 24-08-2006 |
| US 2010137179 | A1 | 03-06-2010 | CN | 101926743 A | 29-12-2010 |
| | | | EP | 2181695 A2 | 05-05-2010 |
| | | | JP | 2010111668 A | 20-05-2010 |
| | | | US | 2010137179 A1 | 03-06-2010 |
| US 2022023160 | A1 | 27-01-2022 | CN | 113164786 A | 23-07-2021 |
| | | | JP WO2020110608 | A1 | 14-10-2021 |
| | | | TW | 202027711 A | 01-08-2020 |
| | | | US | 2022023160 A1 | 27-01-2022 |
| | | | WO | 2020110608 A1 | 04-06-2020 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6063397 A
- EP 1283019 B1
- US 9622944 B
- US 20160367102 A

**Non-patent literature cited in the description**

- Classification of surfactants. **L. OLDENHOVE DE GUERTECHIN.** Handbook of Cosmetic Science and Technology. Marcel Dekker, Inc, 2001, 431-450
- *Oil & Soap,* 1941, vol. 18 (I), 99-102
- **INDA.** NONWOVEN FABRICS HANDBOOK. 1999